Europäisches Patentamt

European Patent Office

Office européen des brevets

(11) Veröffentlichungsnummer: **0 241 593**
**A1**

(12) **EUROPÄISCHE PATENTANMELDUNG**

(21) Anmeldenummer: **86117128.8**

(22) Anmeldetag: **10.12.86**

(51) Int. Cl.4: **A61M 5/14** , A61M 5/00 , F16L 55/14

(30) Priorität: **20.03.86 DE 8607655 U**

(43) Veröffentlichungstag der Anmeldung:
**21.10.87 Patentblatt 87/43**

(84) Benannte Vertragsstaaten:
**AT BE CH DE ES FR GB GR IT LI NL SE**

(71) Anmelder: **INTERMEDICAT GMBH**
**Gerliswilstrasse 74**
**CH-6020 Emmenbrücke(CH)**

(72) Erfinder: **Lesemann, Egon**
**Lindenbergstrasse 28-30**
**D-3508 Melsungen(DE)**
Erfinder: **Rose, Karl-Heinz**
**Bergstrasse 20**
**D-3508 Melsungen-Röhrenfurth(DE)**

(74) Vertreter: **von Kreisler, Alek, Dipl.-Chem. et al**
**Deichmannhaus am Hauptbahnhof**
**D-5000 Köln 1(DE)**

(54) **Rollenklemme für einen Kunststoffschlauch.**

(57) Die Rollenklemme zum Zusammenquetschen eines Schlauchs (21) zum Zwecke der Tropfendosierung weist ein langgestrecktes Gehäuse (10) mit U-förmigem Querschnitt auf. In dem Gehäuse ist eine Rolle (15) verschiebbar, deren Achsstummel (16) in Führungsnuten (14) des Gehäuses geführt sind. Die Rolle (15) quetscht den Schlauch (21) über dem in Längsrichtung ansteigenden Kanalboden (22) zusammen. Zur Erzielung einer hohen Tropfenkonstanz sollen Relaxationen und undefinierte Verformungen und Bewegungen des Schlauchs (21) verhindert werden. Hierzu ist der Kanalboden (22) mindestens teilweise als Schrägfläche ausgebildet, so daß der Schlauchquerschnitt an einer Seite stärker zusammengedrückt wird als an der gegenüberliegenden Seite.

FIG.2

## Rollenklemme für einen Kunststoffschlauch

Die Erfindung betrifft eine Rollenklemme für einen Kunststoffschlauch, mit einem Gehäuse, das einen längslaufenden Kanal für den Schlauch aufweist, und einer Rolle, deren Achse in Führungsnuten der Seitenwände des Kanals geführt ist, wobei der Abstand des Kanalbodens von den Führungsnuten sich in Gehäuselängsrichtung vergrößert und der Kanalboden mindestens in einem Teilbereich seines Querschnitts als Schrägfläche ausgebildet ist.

Bei einer bekannten Schlauchklemme dieser Art (DE-AS 20 43 551) ist im Kanalboden eine Nut mit V-förmigem Querschnitt vorgesehen, deren Tiefe sich in Längsrichtung des Kanals verändert. Der Schlauch ist im vollständig zusammengequetschten Zustand breiter als der Kanalboden, so daß ein Teil der Unterseite des Schlauchs in die Nut hinein verdrängt wird. Der Schlauch liegt nur an den Rändern des Kanalbodens auf. Obwohl durch eine derartige Ausbildung des Kanalbodens das Relaxationsverhalten, d.h. das Rückformungsverhalten, des Schlauchs verbessert werden soll, indem Kaltfließen und ähnliche Ermüdungserscheinungen verhindert werden, erhält man keine definierte Konstanz der durch die Schlauchabquetschung hervorgerufenen Tropfrate, weil die Position, die der Schlauch im Inneren des Kanals und insbesondere in der Nut einnimmt, weitgehend von Zufälligkeiten abhängt. Insbesondere werden unterschiedliche Spannungsverteilungen im Schlauchgefüge erzeugt, weil die Abquetschung ausschließlich an den Umbiegungsstellen erfolgt, während die anderen Bereiche des Schlauchquerschnitts teilweise sogar gestaucht werden.

Weiterhin ist eine Schlauchklemme bekannt (DE-PS 22 42 539), bei der der Kanalboden an einer Seite eine Aussparung aufweist, die sich nach unten sowie in seitlicher Richtung erstreckt und unter Bildung eines Überhanges seitlich über die Innenseite des Kanals hinausragt. Auch hierbei ist die Breite des zusammengedrückten Schlauchs größer als die Bodenfläche des Kanals, so daß ein Teil des Schlauchquerschnitts in die Aussparung hinein ausweicht und dort einen freien Rohrquerschnitt bildet. Auch bei dieser Schlauchklemme ist die Einnahme einer definierten Position des Schlauchquerschnitts nicht sichergestellt, weil der Schlauch in undefinierter Weise in die Aussparung hinein ausweichen kann.

Der Erfindung liegt die Aufgabe zugrunde, eine Schlauchklemme der eingangs genannten Art zu schaffen, bei der ein definiertes Abquetschen des Schlauchs erfolgt und undefinierte Relaxationen weitgehend vermieden werden, so daß die eingestellte Tropfrate sich nachträglich nicht, bzw. nicht wesentlich, verändert.

Zur Lösung dieser Aufgabe ist erfindungsgemäß vorgesehen, daß die Schrägfläche bis zu mindestens einer der Seitenwände reicht und, bezogen auf die Umfangsfläche der Rolle, einen Neigungswinkel von weniger als 5° hat.

Bei der erfindungsgemäßen Rollenklemme, die insbesondere für medizinische Flüssigkeitssysteme, wie z.B. Infusions-oder Transfusionsgeräte, benutzt wird, ist der Kanalboden weder stufenförmig gestaltet noch mit einer Ausweichnut versehen. Vielmehr wird durch die Schrägfläche, die einen kleinen Neigungswinkel hat, erreicht, daß der Schlauch an der einen Seite seines Querschnitts etwas stärker zusammengedrückt wird als an der entgegengesetzten Seite, so daß sich an derjenigen Seite, an der die Zusammenquetschung weniger stark ist, ein definierter Durchlaß ausbildet, ohne daß der Schlauch sich in undefinierter Weise im Kanal bewegt oder verdrängt wird. Die Durchflußöffnung des Schlauchs wird in der Endphase des Abquetschens von einer Seite des Schlauchquerschnitts bis zur gegenüberliegenden Seite zunehmend verringert, ohne dem Schlauch durch Mulden oder Ausformungen des Kanalbodens irgendwelche Ausweichmöglichkeiten zu geben. Dadurch behält der Schlauch seine definierte Lage, die er während des Vorschiebens der Rolle eingenommen hat, bei. Da andererseits kein Bereich der Umfangsfläche des Schlauchs vorhanden ist, der nicht einer mechanischen Preßwirkung ausgesetzt wäre, können unbeabsichtigte Rückbildungen des Schlauchs nur in ganz geringem Maße auftreten.

Der Neigungswinkel der Schrägfläche (quer zur Längsrichtung des Kanals) beträgt vorzugsweise etwa 1°. Diese geringfügige Neigung reicht aus, um sicherzu stellen, daß der Schlauch an einem Ende bereits vollständig zusammengequetscht ist, d.h. keinen Durchgang mehr aufweist, während an der gegenüberliegenden Seite noch ein schmaler Durchlaß vorhanden ist. Ein solches definiertes Zusammendrücken des Schlauchs von einer Seite bis zur gegenüberliegenden Seite bewirkt das Entstehen eines definierten Durchlaßquerschnittes bei jeder Stellung der Rolle.

Gemäß einer bevorzugten Ausführungsform der Erfindung ist vorgesehen, daß die Umfangsfläche der Rolle die gesamte Breite des Kanalbodens einnimmt und daß die Breite des zusammengequetschten Schlauches, dessen Lumen zu einem Strich verformt ist, der Breite des Kanalbodens entspricht. Die Umlenkstellen des Schlauchs stoßen im zusammengequetschten Zustand an die Seitenwände des Kanals an und der Schlauch wird flach zusammengequetscht, so daß zwei übereianderliegende Schlauchschichten entstehen, die beide im wesentlichen ebenflächig sind.

Gemäß einer bevorzugten Weiterbildung der Erfindung ist vorgesehen, daß der Übergang vom Kanalboden zu der Seitenwand an dem höher liegenden Rand des Kanalbodens bogenförmig mit einem etwa der Außenkrümmung des vollständig zusammengequetschten Schlauchs entsprechenden Radius ausgebildet ist, während der Übergang an der entgegengesetzten Seite scharfkantig ist, d.h. etwa rechtwinklig. Dadurch wird das Entstehen örtlicher Spannungsspitzen, die zu Kaltfließen führen könnten, im Schlauch vermieden.

Im folgenden werden unter Bezugnahme auf die Zeichnungen Ausführungsbeispiele der Erfindung näher erläutert.

Es zeigen:

Fig. 1 einen Längsschnitt durch die Schlauchklemme entlang der Linie I-I von Fig. 2, wobei der Schlauch und die Rolle gestrichelt dargestellt sind,

Fig. 2 einen Querschnitt durch die Schlauchklemme entlang der Linie II-II von Fig. 1,

Fig. 3 Draufsichten verschiedener Schlauchklemmen, wobei die Schrägflächen in Längsrichtung schraffiert sind, und

Fig. 4 Querschnitte durch die verschiedenen Schlauchklemmen der Fig. 3 entlang der Linie IV-IV.

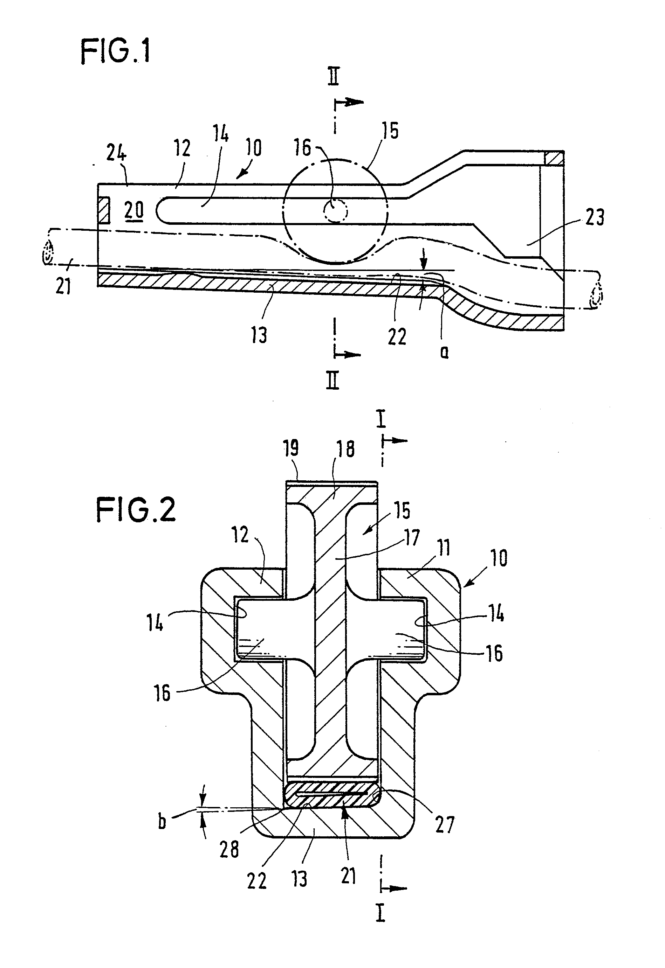

Die Schlauchklemme nach den Figuren 1 und 2 weist ein langgestrecktes Gehäuse mit U-förmigem Querschnitt auf, das an der Oberseite offen ist. Die Seitenwände 11, 12 sind an ihren unteren Enden durch die Bodenwand 13 verbunden. An den Innenseiten der Seitenwände 11, 12 befinden sich parallele Führungsnuten 14 zur Führung der Rolle 15. Die Rolle 15 weist nach entgegengesetzten Seiten abstehende Achsstummel 16 auf, die in die Nuten 14 hineinragen. Die Achsstummel 16 stehen von der Mittelscheibe 17 ab, die außen von einem verbreiterten Umfangsring 18 begrenzt wird. Die Umfangsfläche des Umfangsringes 18 ist mit einer Riffelung 19 versehen.

Die Seitenwände 11 und 12 bilden zusammen mit der Bodenwand 13 einen längslaufenden Kanal 20, in dem die Rolle 15 in Längsrichtung bewegbar ist und aus dessen offener Oberseite die Rolle herausragt. Durch den Kanal 20 führt in Längsrichtung der Schlauch 21 aus PVC hindurch. Der Kanalboden 22 verläuft in Längsrichtung - schräg unter einem Winkel a von etwa 2° in bezug auf die Führungsnuten 14, d.h. sein Abstand von den Führungsnuten 14 vergrößert sich zum Freigabeende 23 hin. Am Freigabeende 23 erweitern sich die Führungsnuten 14, so daß die Rolle 15 dort den Schlauch nicht mehr abquetscht. Am Abquetschende 24 hat der Kanalboden 22 den geringsten Abstand von den Führungsnuten 14. Rollenklemmen mit den bisher beschriebenen Merkmalen sind bekannt.

Wie Figur 2 zeigt, ist der Kanalboden 22 - im Querschnitt gesehen - als Schrägfläche ausgebildet, deren Neigungswinkel b etwa 1° beträgt. Der Kanalboden 22 hat also am linken Rand einen größeren Abstand von der Umfangsfläche 18 als am rechten Rand, so daß bei zunehmender Bewegung der Rolle in Richtung auf das Quetschende 24 (Figur 1) der Schlauch 21 an seiner einen Seite stets etwas stärker zusammengequetscht ist als an seiner anderen Seite. Dies führt dazu, daß sich eine Rest-Durchflußöffnung nur an einer Seite ausbildet (gemäß Figur 2 an der linken Seite), während an der gegenüberliegenden Seite der Schlauch bereits vollständig gequetscht ist, d.h. seine Innenwandteile ohne jegliche Durchflußöffnung abdichtend gegeneinadergedrückt sind.

Die Breite des Kanals 20 ist auf den Schlauchdurchmesser und die Wandstärke des Schlauchs 21 so abgestimmt, daß der Schlauch im vollständig zusammengedrückten Zustand die Breite des Kanalbodens 22 voll einnimmt, während die Schlauchöffnung zu einem im wesentlichen geraden Strich verformt ist.

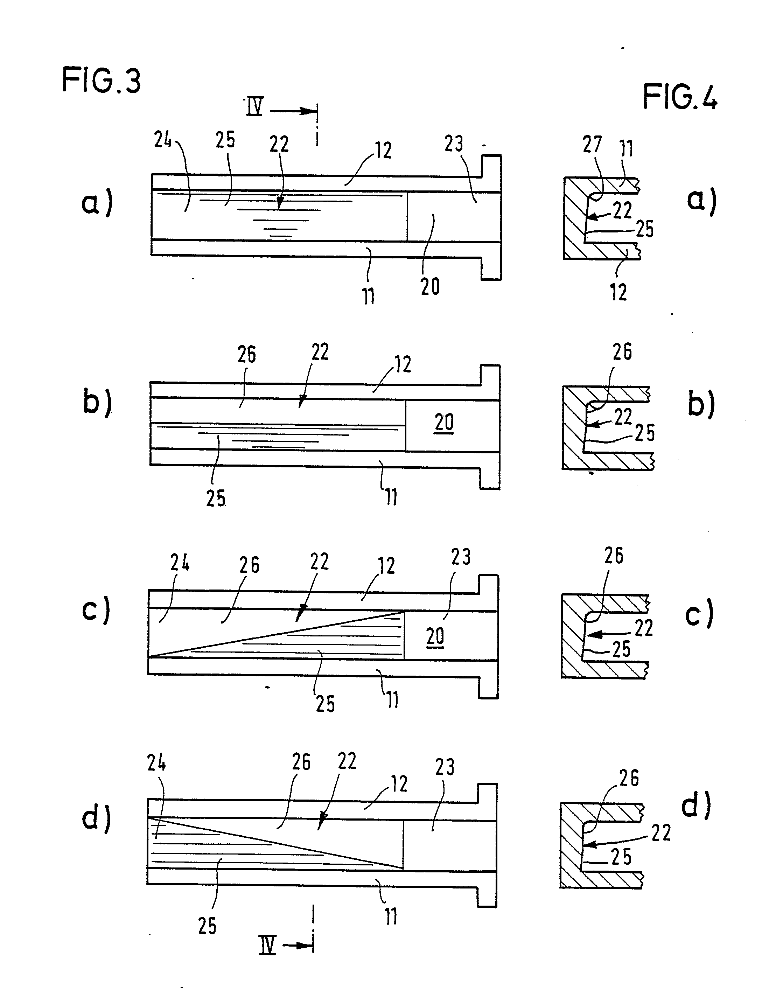

Figuren 3a und 4a zeigen den Kanalboden 22, der über seine gesamte Breite als ebene Schrägfläche 25 ausgebildet ist, entsprechend dem Ausführungsbeispiel der Figuren 1 und 2.

Bei dem Ausführungsbeispiel der Figuren 3b und 4b ist der Kanalboden 22 nur in seiner einen Hälfte als Schrägfläche 25 ausgebildet, während die andere Hälfte eine im Querschnitt parallel zur Umfangswand 18 der Rolle 15 verlaufende Parallelfläche 26 ist. Die Schrägfläche 25 und die Parallelfläche 26 verlaufen in Längsrichtung des Gehäuses 10 unter dem Winkel a (Figur 1) zu den Führungsnuten 14; die Schrägfläche 25 ist zusätzlich quer zur Längsrichtung des Gehäuses unter dem Winkel b geneigt. Jede der Flächen 25, 26 erstreckt sich annähernd über die Hälfte der Breite des Kanals 20.

Gemäß Figuren 3c und 4c verläuft die Knicklinie, an der die Parallelfläche 26 in die Schrägfläche 25 übergeht, schräg zur Längsrichtung des Kanals 20, wobei die Breite der Schrägfläche 25 am Freigabeende 23 die gesamte Kanalbreite einnimmt und sich zum Verschlußende 24 hin stetig verringert.

Bei dem Ausführungsbeispiel der Figuren 3d und 4d nimmt dagegen die Breite der Schrägfläche 25 vom Freigabeende 23 aus in Richtung auf das Verschlußende 24 zu. Diese Variante ist gegenüber derjenigen der Figuren 3c und 4c zu bevorzugen, weil die Schrägfläche 25 gerade im Quetschustand im Hinblick auf eine freie Einstellung der Materialspannungen zweckmäßig ist. Bei nur wenig verformtem Schlauch kann dagegen die Parallelfläche 26 günstiger sein.

Bei allen Ausführungsbeispielen ist die Ecke 27 zwischen der Bodenwand 22 und der Innenseite der Seitenwand 11 an der höchsten Stelle der Bodenwand abgerundet. Der Abrundungsradius der Ecke 27 entspricht der Außenkrümmung der Umbiegung des Schlauchs 21 im vollständig zusammengedrückten Zustand. Die gegenüberliegende Ecke 28 ist dagegen spitz zulaufend, so daß der Schlauch sich in sie hinein verformen kann. Diese Gestaltung der Ecken 27, 28 trägt zu einer vorteilhaften Spannungsverteilung im Schlauchquerschnitt bei.

## Ansprüche

1. Rollenklemme für einen Kunststoffschlauch, mit einem Gehäuse (10), das einen längslaufenden Kanal (20) für den Schlauch (21) und eine Rolle (15), deren Achse in Führungsnuten (14) der Seitenwände (11, 12) des Kanals (20) geführt ist, wobei der Abstand des Kanalbodens (22) von den Führungsnuten (14) sich in Gehäuselängsrichtung vergrößert und der Kanalboden (22) mindestens in einem Teilbereich seines Querschnitts als Schrägfläche (25) ausgebildet ist,
**dadurch gekennzeichnet,**
daß die Schrägfläche (25) bis zu mindestens einer der Seitenwände (12) reicht und, bezogen auf die Umfangsfläche (18) der Rolle (15), einen Neigungswinkel (b) von weniger als 5° hat.

2. Rollenklemme nach Anspruch 1, dadurch gekennzeichnet, daß der Neigungswinkel (b) der Schrägfläche ca. 1° beträgt.

3. Rollenklemme nach Anspruch 1 oder 2, dadurch gekennzeichnet, daß die Umfangsfläche (18) der Rolle (15) die gesamte Breite des Kanalbodens überdeckt und daß die Breite des zusammengequetschten Schlauchs (21), dessen Lumen zu einem Strich verformt ist, der Breite des Kanalbodens (22) entspricht.

4. Rollenklemme nach einem der Ansprüche 1 bis 3, dadurch gekennzeichnet, daß der Übergang (27) vom Kanalboden (22) zu der Seitenwand (11) an dem höher liegenden Rand des Kanalbodens (22) bogenförmig mit einem etwa der Außenkrümmung des vollständig zusammengequetschten Schlauchs (21) entsprechenden Radius ausgebildet ist, während der Übergang an der entgegengesetzten Seite - scharfkantig (28) ist.

5. Rollenklemme nach einem der Ansprüche 1 bis 4, dadurch gekennzeichnet, daß der gesamte Kanalboden (22) im Quetschbereich des Schlauchs als ebene Schrägfläche (25) ausgebildet ist.

6. Rollenklemme nach einem der Ansprüche 1 bis 4, dadurch gekennzeichnet, daß die Schrägfläche (25) etwa die Hälfte des Kanalquerschnitts einnimmt und über die gesamte Länge im wesentlichen konstante Breite hat.

7. Rollenklemme nach einem der Ansprüche 1 bis 4, dadurch gekennzeichnet, daß die Breite der Schrägfläche (25) sich in Längsrichtung stetig verändert.

8. Rollenklemme nach Anspruch 7, dadurch gekennzeichnet, daß die Breite der Schrägfläche (25) zum Verschlußende (24) des Kanals (20) hin zunimmt.

FIG.1

FIG.2

FIG.3

FIG.4

## EINSCHLÄGIGE DOKUMENTE

EP 86117128.8

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 855 572 (K.E.BECKER)<br>* Gesamt; insbesondere Fig. 8; Seite 15, 2. Absatz * | 1 | A 61 M 5/14<br>A 61 M 5/00<br>F 16 L 55/14 |
| A | CH - A - 530 209 (AM. HOSPITAL S. C.)<br>* Gesamt; insbesondere Fig. 3, 7-9; Spalte 4, Zeile 10 - Spalte 5, Zeile 17 * | 1 | |
| D,A | DE - B2 - 2 043 551 (M.ADELBERG)<br>* Gesamt; insbesondere Fig. 1-4; Spalte 3, Zeile 52 - Spalte 5, Zeile 25 * | 1 | |
| A | DE - A1 - 2 512 588 (TERUMO CORP.)<br>* Gesamt; insbesondere Fig. 4-6; Seite 6, 2. und 3. Absatz * | 1 | RECHERCHIERTE SACHGEBIETE (Int. Cl.4)<br><br>A 61 M 5/00<br>F 16 L 55/00 |
| D,A | DE - A - 2 242 539 (TRANSCODAN)<br>* Gesamt; Fig. 2,7-10 * | 1 | |

Der vorliegende Recherchenbericht wurde für alle Patentansprüche erstellt.

| Recherchenort | Abschlußdatum der Recherche | Prüfer |
|---|---|---|
| WIEN | 23-07-1987 | LUDWIG |